(19) Europäisches Patentamt / European Patent Office / Office européen des brevets

(11) **EP 2 854 761 B1**

(12) **FASCICULE DE BREVET EUROPEEN**

(45) Date de publication et mention de la délivrance du brevet:
**01.05.2019 Bulletin 2019/18**

(21) Numéro de dépôt: **13726226.7**

(22) Date de dépôt: **01.06.2013**

(51) Int Cl.:
***A61K 9/16*** *(2006.01)*    ***A61K 9/20*** *(2006.01)*

(86) Numéro de dépôt international:
**PCT/EP2013/061332**

(87) Numéro de publication internationale:
**WO 2013/178817 (05.12.2013 Gazette 2013/49)**

(54) **COMPOSITIONS VÉTÉRINAIRES ORALES APPÉTENTES**

SCHMACKHAFTE ORALE VETERINÄRMEDIZINISCHE ZUSAMMENSETZUNGEN

PALATABLE ORAL VETERINARY COMPOSITIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012 FR 1255122**

(43) Date de publication de la demande:
**08.04.2015 Bulletin 2015/15**

(73) Titulaire: **Ceva Santé Animale**
**33500 Libourne Cedex (FR)**

(72) Inventeurs:
• **GUIMBERTEAU, Florence**
**33450 Montussan (FR)**
• **PEYROT, Laurence**
**33500 Les Billaux (FR)**

(74) Mandataire: **Tezier Herman, Béatrice et al**
**Cabinet Becker & Associés**
**25, rue Louis le Grand**
**75002 Paris (FR)**

(56) Documents cités:
**WO-A2-01/87284     FR-A1- 2 917 975**

• **KAMBARA ATSUSHI ET AL: "Combined effects of low-dose oral spironolactone and captopril therapy in a rat model of spontaneous hypertension and heart failure", JOURNAL OF CARDIOVASCULAR PHARMACOLOGY, RAVEN PRESS, NEW YORK, NY , vol. 41, no. 6 1 juin 2003 (2003-06-01), pages 830-837, XP008152176, ISSN: 0160-2446 Extrait de l'Internet: URL:http://journals.lww.com/cardiovascular pharm/Fulltext/2003/06000/Combined_Effects _of_Low_dose_Oral_Spironolactone.2.aspx**
• **BARR C S ET AL: "Effects of adding spironolactone to an angiotensin-converting enzyme inhibitor in chronic congestive heart failure secondary to coronary artery diseases", AMERICAN JOURNAL OF CARDIOLOGY, CAHNERS PUBLISHING CO., NEWTON, MA, US, vol. 76, no. 17, 15 décembre 1995 (1995-12-15), pages 1259-1265, XP008087876, ISSN: 0002-9149, DOI: 10.1016/S0002-9149(99)80353-1**
• **BIE P ET AL: "Enhanced atrial peptide natriuresis during angiotensin and aldosterone blockade in dogs", AMERICAN JOURNAL OF PHYSIOLOGY,, vol. 258, no. 5 Pt 2, 1 mai 1990 (1990-05-01), pages R1101-R1107, XP008087870, ISSN: 0002-9513**

EP 2 854 761 B1

# EP 2 854 761 B1

## Description

La présente invention telle que revendiquée a pour objet des compositions vétérinaires orales appétentes à base d'un ou plusieurs principes actifs pharmaceutiques choisis parmi la spironolactone et/ou le benazépril, ayant une odeur et/ou un gout répulsif pour les animaux, ainsi qu'un procédé de préparation de préparation desdites compositions vétérinaires orales.

L'administration de principes actifs pharmaceutiques dont le gout et/ou l'odeur sont répulsives est toujours problématique dans le cas des animaux, et requiert généralement l'ajout d'un agent d'appétence de sorte à masquer le mauvais gout ou l'odeur désagréable de ces substances pharmaceutiques et ainsi déjouer l'odorat développé des animaux.

Les agents d'appétence utilisés sont en général des arômes, des levures, protéines, des hydrolysats de protéines, de la gélatine, etc.... et sont le plus souvent des produits hygroscopiques avec une eau résiduelle libre élevée supérieure à 3%. Des problèmes majeurs d'incompatibilité et d'instabilité se posent donc lorsqu'il s'agit de les formuler avec des principes actifs pharmaceutiques sensibles à l'humidité.

Pour régler ce problème, l'homme de l'art s'est orienté vers une séparation physique du principe actif pharmaceutique et de l'agent d'appétence par exemple par enrobage du ou des principes actifs par un film protecteur. Cet enrobage permet en outre de masquer le goût du principe actif pharmaceutique, limitant ainsi les quantités de facteur d'appétence à incorporer. Cette stratégie nécessite la mise en place d'une étape supplémentaire dans le procédé de fabrication ainsi qu'un cout supplémentaire associé.

La Demanderesse a découvert qu'il suffisait d'obtenir un taux d'eau libre résiduelle inférieure à une limite de 1,5% pour surmonter les problèmes d'incompatibilité entre l'agent d'appétence dit « humide » ou hygroscopique et le principe actif pharmaceutique, et ainsi d'obtenir une stabilité optimale après formulation de ce dernier.

## Résumé de l'invention

La présente invention a donc pour objet un procédé de préparation d'une composition vétérinaire orale appétente, comprenant une étape de mélange d'un ou plusieurs principes actifs pharmaceutiques choisis parmi la spironolactone et/ou le benazépril, ayant une odeur et/ou un gout répulsif pour les animaux avec au moins un agent d'appétence, hygroscopique et une étape de post-séchage du mélange de sorte à obtenir une quantité d'eau libre résiduelle dans le mélange post-séchage comprise entre 0 et 1,5% en poids.

La teneur en eau libre résiduelle est préférentiellement déterminée par la méthode de titrage de Karl Fisher après déduction éventuelle de la quantité d'eau liée (ou eau de cristallisation) des ingrédients de la composition :

$$\text{Eau libre résiduelle} = \text{eau totale Karl Fisher} - \text{eau liée}$$

La présente invention concerne également une composition vétérinaire orale appétente comprenant au moins un principe actif pharmaceutique ayant une odeur et/ou un gout répulsif pour les animaux et au moins un agent d'appétence, le pourcentage d'eau libre résiduelle de ladite composition vétérinaire étant compris entre 0 et 1,5% en poids.

## Brève description des Figures

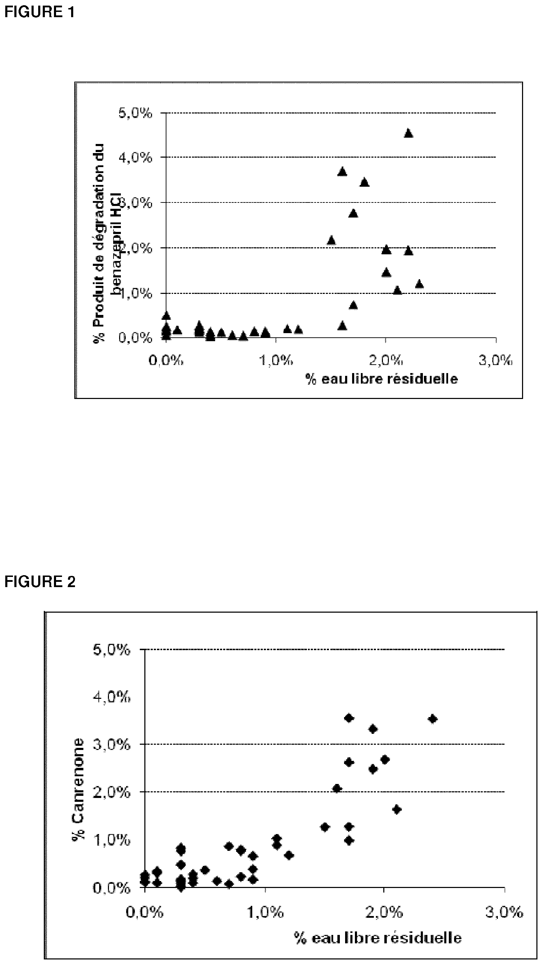

Figure 1 : est un graphe montrant l'influence de l'eau libre résiduelle sur la quantité de produits de dégradation générés au cours du stockage d'une composition de benazepril HCl.
Figure 2 : est un graphe montrant l'influence de l'eau libre résiduelle sur la quantité de produits de dégradation (canrenone) générée au cours du stockage d'une composition de spironolactone.

## Description détaillée de l'invention

La présente invention concerne donc un procédé de préparation d'une composition vétérinaire orale appétente, comprenant (i) une étape de mélange d'un ou plusieurs principes actifs pharmaceutiques choisis parmi la spironolactone et/ou le benazépril, ayant une odeur et/ou un gout répulsif pour les animaux avec au moins un agent d'appétence, hygroscopique ayant une teneur en eau libre résiduelle supérieure à 1,5% en poids par rapport au poids total de la composition, et (ii) une étape de post-séchage du mélange de sorte à ce que l'eau libre résiduelle du mélange après l'étape de post-séchage soit comprise entre 0 à 1,5%, par rapport au poids total de la composition.

L'eau libre résiduelle est préférentiellement déterminée par le titrage de Karl Fisher en déduisant la quantité d'eau liée éventuellement présente dans la composition.

La présente invention est en effet basée sur la découverte de l'existence d'une valeur critique de l'eau libre résiduelle de la composition vétérinaire orale au dessous de laquelle les problèmes d'incompatibilité entre l'agent d'appétence hygroscopique et l'actif pharmaceutique, et d'instabilité de ce dernier sont résolus. Plus précisément l'agent actif pharmaceutique choisis parmi la spironolactone et/ou le benazépril, reste stable après formulation avec l'agent d'appétence lorsque l'eau libre résiduelle obtenue après séchage était inférieure à 1.5%.

La méthode de titrage de Karl Fisher est une méthode efficace, rapide, fiable destinée à la détermination de la quantité d'eau dans une grande variété d'échantillons ou de principes actifs pharmaceutiques et sur une large gamme de concentration. Elle est basée sur la réaction d'oxydation du dioxyde de soufre par l'iode dissous dans du méthanol et une base telle que la pyridine, selon la réaction suivante :

$$SO_2 + I_2 + H_2O \rightarrow H_2SO_4 + HI$$

à pH compris entre 5 et 7

Dans cette réaction, l'acide sulfurique $SO_2$ et l'acide iodhydrique $I_2$ ne réagissent qu'en présence d'eau. L'iode participant à la réaction est générée directement dans la cellule de titrage par une oxydation électrochimique de l'iodure jusqu'à ce que de l'iode non réactive soit détectée. Le point final se détermine par colorimétrie ou ampérométrie. Cette méthode est notamment utilisée pour s'assurer de la régularité lors de la fabrication des médicaments lyophilisés.

De nombreux appareillages existent sur le marché afin de faciliter un tel dosage. On peut citer à titre d'exemples, le titrateur volumétrique Karl Fischer TIM550® commercialisé par la société Radiometer analytical, le Titrino KF®, le Titrando KF®, ou le Coulomètre KF®.

L'eau liée ou eau de cristallisation peut être déterminée de manière expérimentale à l'aide par exemple d'une analyse thermogravimétrique (mesure directe des variations de masse en fonction de la température). Ainsi, l'eau libre est éliminée à des températures inférieures à environ 100°C alors que l'eau liée n'est éliminée qu'à des températures supérieures à 100°C. L'eau liée ou eau de cristallisation peut être également déterminée de manière théorique, lorsque le degré d'hydratation d'une molécule est connu.

Selon le procédé de la présente invention, la teneur en eau libre résiduelle de l'agent d'appétence hygroscopique doit être inférieur à 1,5% en poids par rapport au poids total de la composition.

Le procédé selon la présente invention est particulièrement utile pour la formulation de compositions vétérinaires orales appétentes comprenant un ou plusieurs principes actifs pharmaceutiques ayant une odeur et/ou un gout répulsif Ceux-ci sont choisis parmi la spironolactone et/ou le benazépril. Le procédé est également avantageux lorsque lesdits principes actifs pharmaceutiques sont instables et sensibles à l'humidité.

Parmi les antagonistes de l'aldostérone, on cite la spironolactone.

Parmi les inhibiteurs d'enzyme de conversion de l'angiotensine, on cite le bénazépril et ses sels et les esters.

Les principes actifs pharmaceutiques sont choisis parmi la spironolactone et/ou le benazépril.

Ces compositions sont particulièrement utiles pour traiter les animaux non humains atteints d'insuffisance cardiaque comme les cardiopathies congénitales ou les cardiopathies acquises tel que cela est décrit notamment dans la publication internationale WO2009/000843.

Dans la publication FR2917975 une composition vétérinaire est décrite comprenant du Benazépril et/ou du Spironolactone sous forme de comprimé ou de granules.

La présente invention a donc également pour objet les compositions vétérinaires telles que précédemment décrites, pour leur utilisation dans le traitement et/ou la prévention de l'insuffisance cardiaque chez les animaux non humains.

Les compositions vétérinaires selon la présente invention peuvent alors par exemple comprendre des doses quotidiennes thérapeutiquement efficaces d'un antagoniste du récepteur de l'aldostérone, la spironolactone comprises entre environ 0,88 et 5 mg/kg/jour (de préférence d'environ 2 mg/kg/jour) et des doses d'inhibiteurs d'enzyme de conversion de l'angiotensine, le benazépril, comprises entre 0,1 à 0,6 mg/kg/jour (de préférence d'environ 0,25 mg/kg/jour).

Les agents d'appétence susceptibles d'être utilisés dans le procédé selon la présente invention sont également bien connus dans le domaine. Ceux-ci sont par exemple des arômes, des levures, protéines, des hydrolysats de protéines, de la gélatine, de l'amidon ou amidon pré-gélatinisé, des composés à base d'acide phosphorique, le pyrophosphate disodique, pyrophosphate tétrasodique, etc...

L'étape de post-séchage peut être réalisée par tous moyens bien connus dans le domaine, par exemple en plaçant la composition sous vide partiel pour accélérer le séchage, ou encore par ajout d'un agent dessiccateur ou desséchant, tel que par exemple le gel de silice ou silicagel, les agents desséchant du type chlorure de calcium anhydre, les tamis moléculaires, les tunnels de séchage, les systèmes de séchage par micro-onde, etc...

De préférence, les compositions sont fabriquées et/ou conditionnées sous humidité réduite.

Selon le procédé de l'invention, la teneur en eau libre résiduelle après l'étape de séchage est entre 0 et 1,5% en poids par rapport au poids total de la composition.

Cette teneur en eau résiduelle est préférentiellement déterminée par le titrage de Karl Fisher duquel on déduit la quantité d'eau liée éventuellement présente dans les ingrédients de la composition.

Les compositions vétérinaires orales obtenues par le procédé décrit précédemment présentent des propriétés supérieures d'appétence, celles-ci sont de préférence supérieures à 50% de prise spontanée.

Lesdites compositions vétérinaires orales peuvent se présenter sous toutes les formes appropriées pour une administration par voie orale. Elles peuvent donc être par exemple sous forme solide ou semi-solide, de poudres, de comprimés, de capsules, de granules, de dragées, de gélules, de sprays, de cachets, de pilules, de tablettes, ou de pâtes.

Les compositions vétérinaires orales obtenues par le procédé décrit précédemment présentent par ailleurs une meilleure stabilité lors du stockage. Elles sont en effet stables pendant au moins 24 mois sans condition particulière de stockage au sens des recommandations publiées le 20 mai 1999 par le comité VICH sous le titre VICH GL3 (Stability 1) « Stability Testing of New Veterinary Drug Substances and Médicinal Products ». En effet, comme cela a été démontré dans les exemples ci-après, moins de 3% est dégradé après 24 mois de stockage à des températures de 25-30°C et sous 60-65% d'humidité relative (RH).

Selon un second aspect, la présente invention a pour objet des compositions vétérinaires orales appétentes, comprenant un ou plusieurs principes actifs pharmaceutiques, choisis parmi la spironolactone et/ou le benazépril, ayant une odeur et/ou un gout répulsif pour les animaux, au moins un agent d'appétence hygroscopique, et éventuellement des excipients ayant un pourcentage d'eau libre résiduelle supérieur à 1,5%. La teneur en eau libre résiduelle dans les compositions vétérinaires telles que revendiquées, est comprise entre 0 et 1,5% en poids.

Les principes actifs pharmaceutiques et les agents d'appétence sont tels que précédemment décrits et tels que revendiquées.

Les compositions vétérinaires orales selon cet aspect de la présente invention sont particulièrement avantageuses puisqu'elles permettent d'obtenir de manière simple une appétence supérieure à 50% de prise spontanée. Elles présentent par ailleurs des caractéristiques de stabilité, lors de stockage à longs termes, supérieures aux compositions dont l'eau libre résiduelle est supérieure à 1,5%.

Egalement, les compositions selon l'invention peuvent comprendre tout autre excipient pharmaceutiquement acceptable, tel que les sucres (lactose, lactose monohydrate, saccarose, dextrose, glucose, etc..), la cellulose ou les amidons, un agent de désintégration, les lubrifiants, les liants, les diluants, les antioxydants, des agents d'écoulement, des agents complexants, des agents de conservation, des colorants, ou des agents tampons etc...

Comme lubrifiant, on peut utiliser l'acide stéarique, le stéarate de magnésium, la silice colloïdale ou, par exemple, le tribéhénate de glycérol. Comme agent de désintégration, on peut utiliser la croscarmellose, la crospovidone, les dérivés d'amidons. Des exemples de liants englobent la méthylcellulose, l'hydroxyéthylcellulose, la gomme de xanthane, la povidone, la cellulose microcristalline, etc....

## EXEMPLES

### Exemple 1 : Préparation de compositions vétérinaires appétentes de spironolactone.

Des comprimés de spironolactone ont été préparés selon un procédé en deux étapes principales : granulation humide suivie d'une compression.

Etape 1 : granulation

Lors de l'étape de granulation, les ingrédients listés dans le Tableau 1 ci-dessous ont été mélangés à sec, granulés à l'eau dans un granulateur à haut cisaillement DIOSNA puis séchés à l'étuve.

Tableau 1 :

| Ingrédients de granulation | Composition en % (m/m) |
|---|---|
| Spironolactone | 20 |
| Lactose monohydrate | 40 |
| Cellulose microcristalline | 25 |
| Crospovidone | 5 |
| Povidone | 10 |

Etape 2 : compression

Les granulés obtenus à l'issue de l'étape 1 ont été ensuite mélangés avec des excipients de compression et les agents appétents puis comprimés à l'aide d'une presse FROGERAIS. La composition finale des comprimés de 250 mg est donnée dans le Tableau 2 ci-dessous.

Tableau 2 :

| Ingrédients de compression | Composition en % (m/m) |
|---|---|
| Granulés de Spironolactone | 60 |
| Arome artificiel foie de porc | 15 |
| Sucre compressible | 20 |
| Crospovidone | 4 |
| Stearate de magnésium | 1 |

**Exemple 2 : Préparation de compositions vétérinaires appétentes de benazepril**

Des comprimés de benazepril HCl sont préparés selon un procédé en deux étapes principales : granulation humide suivi d'une compression.

Etape 1 : granulation

Lors de l'étape de granulation, les ingrédients listés dans le Tableau 3 ci-dessous ont été mélangés à sec, granulés à l'éthanol dans un granulateur à haut cisaillement DIOSNA puis séchés sous vide.

Tableau 3 :

| Ingrédients de granulation | Composition en % (m/m) |
|---|---|
| Benazepril HCl | 2 |
| Lactose monohydrate | 60 |
| Cellulose microcristalline | 30 |
| Crospovidone | 3 |
| Povidone | 5 |

Etape 2 : compression

Les granulés obtenus à l'issue de l'étape 1 ont été ensuite mélangés avec des excipients de compression et les agents appétents puis comprimés à l'aide d'une presse FROGERAIS. La composition finale des comprimés de 200 mg est donnée dans le Tableau 4 ci-dessous.

Tableau 4 :

| Ingrédients de compression | Composition en % (m/m) |
|---|---|
| Granulés de benazepril HCl | 50 |
| Arome artificiel poulet | 20 |
| Sucre compressible | 25 |
| Crospovidone | 4 |
| Stéarate de magnésium | 1 |

Le lactose monohydrate contient 5% d'eau liée. Les comprimés de l'Exemple 2 contiennent donc 1,5% d'eau liée apportée par le lactose monohydrate.

**Exemple 3 : Préparation de compositions vétérinaires appétentes de spironolactone et de benazepril HCl**

Des comprimés combo contenant à la fois de la spironolactone et du benazepril HCl ont été préparés selon un procédé en deux étapes.

La composition des comprimés combo est donnée dans le Tableau 5 ci-dessous :

Tableau 5 :

| Ingrédient | Composition en % (m/m) |
|---|---|
| Granulés de benazepril HCl et de spironolactone | 66.2 |
| Arome artificiel boeuf | 20.0 |
| Sucre compressible | 10.0 |
| Crospovidone | 3.0 |
| Stearate de magnésium | 0.8 |

**Exemple 4 : Tests de stabilité et humidité libre résiduelle**

Les comprimés de l'Exemple 3 ont été placés dans des piluliers en polyéthylène haute densité (PEHD) et stockés dans des enceintes climatiques à 25°C/60% RH et 30°C/65% RH. Des analyses ont été réalisées régulièrement pendant 2 ans. Les résultats sont donnés dans le Tableau 6 ci-dessous.

Tableau 6 :

| Stockage | Test | Point de stabilité | | | | | | |
|---|---|---|---|---|---|---|---|---|
| | | 0 | 3 mois | 6 mois | 9 mois | 12 mois | 18 mois | 24 mois |
| Condition stockage 25°C/60% RH | Teneur en eau libre résiduelle (%) | 0.2 | 0.0 | 0.2 | 0.3 | 0.2 | 0.5 | 0.6 |
| | Dosage spironolactone (mg/comprimé) | 40.3 | 40.2 | 39.8 | 39.4 | 39.9 | 39.8 | 39.9 |
| | Produit dégradation spironolactone (%) | 0.2 | 0.3 | 0.4 | 0.5 | 0.5 | 0.7 | 0.9 |
| | Dosage benazepril HCl (mg/comprimé) | 5.05 | 5.01 | 5.02 | 4.95 | 4.96 | 4.92 | 4.99 |
| | Produits dégradation benazepril HCl (%) | <LOQ | 0.2 | 0.4 | 0.4 | 0.4 | 0.7 | 0.7 |
| Condition stockage 30°C/65% RH | Teneur en eau libre résiduelle (%) | 0.2 | 0.2 | 0.2 | 0.5 | 0.4 | 0.3 | 1.0 |
| | Dosage spironolactone (mg/comprimé) | 40.3 | 40 | 39.7 | 39 | 39.9 | 39.8 | 39.7 |
| | Produit dégradation spironolactone (%) | 0.2 | 0.4 | 0.5 | 0.7 | 0.8 | 1.3 | 1.6 |
| | Dosage benazepril HCl (mg/comprimé) | 5.05 | 4.99 | 5.01 | 4.89 | 4.96 | 4.91 | 4.95 |
| | Produits dégradation benazepril HCl (%) | < LOQ | 0.3 | 0.4 | 0.5 | 0.5 | 0.9 | 1.1 |

Les teneurs en impuretés totales restent à des niveaux très faibles (<3%) après 2 ans de stockage lorsque l'humidité libre dans les comprimés demeure faible.

EP 2 854 761 B1

**Exemple 5 : Contre-exemple**

Des comprimés de composition identique à celle de l'Exemple 3 ont été stockés à 25°C/60%RH de manière à avoir des comprimés avec des teneurs en eau libre résiduelle élevées (contre-exemple) ou faibles selon la présente inventions. Les analyses à 3 et 6 mois ont montré que l'évolution des produits de dégradation était élevée lorsque la teneur en eau libre est supérieure au seuil de 1,5%. Sur le long terme, les comprimés ayant une humidité libre résiduelle élevée n'étaient pas suffisamment stables.

Tableau 7 :

| Produit | Test | Point de stabilité | |
|---|---|---|---|
| | | 3 mois | 6 mois |
| Exemple comparatif | Teneur en eau libre résiduelle (%) | 2.1 | 2.5 |
| | Produit dégradation spironolactone (%) | 0.6 | 1.1 |
| | | | |
| Invention | Teneur en eau libre résiduelle (%) | 0.6 | 0.3 |
| | Produit dégradation spironolactone (%) | 0.1 | 0.2 |

**Revendications**

1. Procédé de préparation d'une composition vétérinaire orale appétente, comprenant une étape de mélange d'un ou plusieurs principes actifs pharmaceutiques choisis parmi la spironolactone et/ou le benazépril ayant une odeur et/ou un gout répulsif pour les animaux avec un agent d'appétence, hygroscopique ayant une teneur en eau libre résiduelle supérieure à 1,5% en poids par rapport au poids total de la composition, et une étape de post-séchage du mélange, **caractérisé en ce que** la teneur en eau libre résiduelle de la composition, après l'étape de post-séchage est comprise entre 0 et 1,5% en poids par rapport au poids total de la composition.

2. Procédé selon la revendication 1, **caractérisé en ce que** la composition vétérinaire contient en outre au moins un excipient et/ou un lubrifiant.

3. Procédé selon l'une quelconque des revendications précédentes, **caractérisé en ce que** l'étape de post-séchage est réalisée par l'ajout d'un agent dessiccateur ou d'un desséchant, de préférence l'agent dessiccateur ou desséchant est le gel de silice.

4. Procédé selon l'une quelconque des revendications précédentes, **caractérisé en ce que** la composition vétérinaire orale appétente est stable pendant au moins 24 mois sans condition particulière de stockage.

5. Procédé selon l'une quelconque des revendications précédentes, **caractérisé en ce que** moins de 3% du principe actif pharmaceutique est dégradé après 24 mois de stockage à des températures de 25-30°C et sous 60-65% d'humidité relative.

6. Composition vétérinaire orale appétente, **caractérisée en ce qu'**il comprend un ou plusieurs principes actifs pharmaceutiques choisis parmi la spironolactone et/ou le benazépril ayant une odeur et/ou un gout répulsif pour les animaux, et un agent d'appétence, hygroscopique ayant une teneur en eau libre résiduelle supérieure à 1,5% en poids par rapport au poids total de la composition, et **caractérisée** en ce ladite composition présente une teneur en eau libre résiduelle comprise entre 0 et 1,5% en poids par rapport au poids total de la composition.

7. Composition selon la revendication 6, **caractérisée en ce que** la composition vétérinaire contient en outre au moins un excipient et/ou un lubrifiant.

8. Composition selon la revendication 6 ou 7, **caractérisée en ce que** la composition se présente sous forme solide ou semi-solide, de poudres, de comprimés, de capsules, de granules, de dragées, de gélules, de sprays, de cachets, de pilules, de tablettes, ou de pâtes.

9. Composition vétérinaire selon l'une quelconques des revendications 6 à 8, pour son utilisation dans le traitement et/ou la prévention de l'insuffisance cardiaque chez les animaux non humains.

10. Composition vétérinaire selon la revendication 9, **caractérisée en ce que** la spironolactone est présente en une dose comprise entre environ 0,88 et 5 mg/kg/jour et de préférence d'environ 2 mg/kg/jour.

11. Composition vétérinaire selon la revendication 9, **caractérisée en ce que** le benazepril est présent en une dose comprise entre environ 0,1 et 0,6 mg/kg/jour, et de préférence d'environ 0,25 mg/kg/jour.

**Patentansprüche**

1. Verfahren zur Herstellung einer schmackhaften oralen Veterinärzusammensetzung, umfassend einen Schritt der Mischung eines oder mehrerer pharmazeutischer Wirkstoffe, ausgewählt aus Spironolacton und/oder Benazepril, mit einem für Tiere abstoßenden Geruch und/oder Geschmack mit einem hygroskopischen schmackhaften Mittel mit einem freien Restwassergehalt größer als 1,5 Gew.-% bezogen auf das Gesamtgewicht der Zusammensetzung, und einen Schritt der Nachtrocknung der Mischung, **dadurch gekennzeichnet, dass** der freie Restwassergehalt der Zusammensetzung nach dem Nachtrocknungsschritt zwischen 0 und 1,5 Gew.-% bezogen auf das Gesamtgewicht der Zusammensetzung beträgt.

2. Verfahren gemäß Anspruch 1, **dadurch gekennzeichnet, dass** die Veterinärzusammensetzung außerdem wenigstens einen Arzneimittelträger und/oder ein Gleitmittel enthält.

3. Verfahren gemäß einem der vorhergehenden Ansprüche, **dadurch gekennzeichnet, dass** der Nachtrocknungsschritt durch Zugabe eines Exsikkans oder eines Trockenmittels erfolgt, vorzugsweise das Exsikkans oder Trockenmittel Silicagel ist.

4. Verfahren gemäß einem der vorhergehenden Ansprüche, **dadurch gekennzeichnet, dass** die schmackhafte orale Veterinärzusammensetzung wenigstens 24 Monate ohne spezielle Lagerungsbedingungen stabil ist.

5. Verfahren gemäß einem der vorhergehenden Ansprüche, **dadurch gekennzeichnet, dass** weniger als 3% des pharmazeutischen Wirkstoffes nach einer Lagerung von 24 Monaten bei Temperaturen von 25-30°C und bei 60-65% relativer Luftfeuchtigkeit abgebaut ist.

6. Schmackhafte orale Veterinärzusammensetzung, **dadurch gekennzeichnet, dass** sie einen oder mehrere pharmazeutische Wirkstoffe, ausgewählt aus Spironolacton und/oder Benazepril, mit einem für Tiere abstoßenden Geruch und/oder Geschmack und ein hygroskopisches schmackhaftes Mittel mit einem freien Restwassergehalt größer als 1,5 Gew.-% bezogen auf das Gesamtgewicht der Zusammensetzung umfasst, und **dadurch gekennzeichnet, dass** besagte Zusammensetzung einen freien Restwassergehalt zwischen 0 und 1,5 Gew.-% bezogen auf das Gesamtgewicht der Zusammensetzung aufweist.

7. Zusammensetzung gemäß Anspruch 6, **dadurch gekennzeichnet, dass** die Veterinärzusammensetzung außerdem wenigstens einen Arzneimittelträger und/oder ein Gleitmittel enthält.

8. Zusammensetzung gemäß Anspruch 6 oder 7, **dadurch gekennzeichnet, dass** die Zusammensetzung in fester oder halbfester Form, als Pulver, Tabletten, Kapseln, Granulate, Dragees, Gelatinekapseln, Sprays, Oblatenkapseln, Pillen, Pastillen oder Pasten vorliegt.

9. Veterinärzusammensetzung gemäß einem der Ansprüche 6 bis 8 zur Verwendung in der Behandlung und/oder Prävention der Herzinsuffizienz bei nicht-humanen Tieren.

10. Veterinärzusammensetzung gemäß Anspruch 9, **dadurch gekennzeichnet, dass** Spironolacton in einer Dosis zwischen ungefähr 0,88 und 5 mg/kg/Tag und vorzugsweise ungefähr 2 mg/kg/Tag vorliegt.

11. Veterinärzusammensetzung gemäß Anspruch 9, **dadurch gekennzeichnet, dass** Benazepril in einer Dosis zwischen ungefähr 0,1 und 0,6 mg/kg/Tag und vorzugsweise ungefähr 0,25 mg/kg/Tag vorliegt.

**Claims**

1.  A process for preparation of a palatable oral veterinary composition, comprising a stage of mixing one or more pharmaceutical active substances chosen among spironolactone and/or benazepril having an odor and/or a taste repulsive to animals with a hygroscopic palatabilizing agent having a free residual water content greater than 1.5% by weight relative to the total weight of the composition, and a stage of post-drying the mixture, **characterized in that** the free residual water content of the composition after the post-drying stage is between 0 to 1.5% by weight relative to the total weight of the composition.

2.  The process according to claim 1, **characterized in that** the veterinary composition further contains at least one excipient and/or one lubricant.

3.  The process according to any one of preceding claims, **characterized in that** the post-drying stage is effected by addition of a desiccating agent or a drying agent, preferably the desiccating agent or drying agent is silica gel.

4.  The process according to any one of the preceding claims, **characterized in that** the palatable oral veterinary composition is stable for at least 24 months with no special storage condition.

5.  The process according to any one of the preceding claims, **characterized in that** less than 3% of the pharmaceutical active substance is degraded after 24 months of storage at temperatures of 25-30°C and at 60-65% relative humidity.

6.  A palatable oral veterinary composition **characterized in that** it comprises one or more pharmaceutical active substances chosen among spironolactone and/or benazepril having an odor and/or a taste repulsive to animals, and a hygroscopic palatabilizing agent having a free residual water content greater than 1.5% by weight relative to the total weight of the composition, and **characterized in that** said composition has a free residual water content comprised between 0 and 1.5% by weight relative to the total weight of the composition.

7.  The composition according to claim 6, **characterized in that** the veterinary composition further contains at least one excipient and/or one lubricant.

8.  The composition according to claim 6 or 7, **characterized in that** the composition is in solid or semi-solid form, powders, tablets, capsules, granules, coated tablets, gel capsules, sprays, cachets, pills, lozenges or pastes.

9.  A veterinary composition according to any one of claims 6 to 8, for use in the treatment and/or prevention of cardiac insufficiency in non-human animals.

10. The veterinary composition according to claim 9, **characterized in that** the spironolactone is present in a dose of between about 0.88 and 5 mg/kg/day and preferably about 2 mg/kg/day.

11. The veterinary composition according to claim 9, **characterized in that** the benazepril is present in a dose of between about 0.1 and 0.6 mg/kg/day and preferably about 0.25 mg/kg/day.

FIGURE 1

FIGURE 2

**EP 2 854 761 B1**

## RÉFÉRENCES CITÉES DANS LA DESCRIPTION

### Documents brevets cités dans la description

- WO 2009000843 A

- FR 2917975